# EUROPEAN PATENT APPLICATION

(11) **EP 2 404 596 A1**
(43) Date of publication of application: **11.01.2012**
(21) Application number: 11184021.1
(22) Date of filing: 22.02.2007
(51) Int. Cl.: A61K 31/196, A61K 31/198, A61K 31/20, A61K 31/337, A61K 31/513, A61K 31/53, A61K 31/573, A61K 31/704, A61K 33/36, A61P 35/00, A61P 43/00

(54) **Kit for Cancer Treatment and Pharmaceutical Composition for Cancer Treatment**

(30) Priority: 23.03.2006 JP 2006080691; 13.02.2007 JP 2007032777
(62) Divisional of application: 07737319.9
(71) Applicant: TMRC Co., Ltd., Tokyo 107-0052 (JP)
(72) Inventor: Ekimoto, Hisao, Kita-ku, Tokyo 115-0042 (JP)
(74) Representative: Jones, Helen M.M.

(57) **Abstract**

To provide a kit for cancer treatment and a pharmaceutical composition for cancer treatment that can inhibit the growth of tumors and cancers in mammals more than ever.

The kit for cancer treatment includes a combination of two different drugs in a kit formulation. The first drug contains a synthetic retinoid or a pharmaceutically acceptable organic or inorganic acid addition salt thereof, and the second drug contains a chemotherapeutic agent for cancer treatment. A synthetic retinoid that can be suitably used is a benzoic acid derivative represented by the following formula (I): 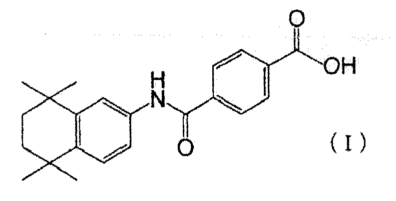 or a pharmaceutically acceptable organic or inorganic acid addition salt thereof.

## Description

### Technical Field

The present invention relates to a kit for inhibiting the growth of tumors and cancers in mammals, and more specifically relates to a kit for cancer treatment and a pharmaceutical composition for cancer treatment for inhibiting the growth of cancers and tumors in mammals, in particular, human and warm-blooded animals.

### Background Art

Cancers are the leading cause of death in animals and human. Many types of chemotherapeutic agents have been shown to be effective against cancer and tumor cells, but unfortunately, many of these agents have a problem that they also destroy normal cells. Despite improvements in the field of cancer treatment, the leading therapies to date are surgery, radiation, and chemotherapy.

Chemotherapeutic approaches are recognized to be effective against cancers that are metastatic or are particularly progressive. The exact mechanism for the action of these chemotherapeutic agents are not always known. Moreover, while some chemotherapeutic agents significantly reduce tumor masses after treatment with such agents, it happens often that they unfortunately cannot be administered again to the same patients when the tumors recurred. In addition, since the width between a therapeutically effective dose and a maximum tolerated dose of chemotherapeutic agents is generally narrow, the administration amount must be determined meticulously.

Today, retinoids are known to be involved in signal transduction systems of immune cells and cause various immune reactions. Recently, it has been reported that retinoids suppress Th1 expression on T cells and enhance Th2 expression (Non-Patent Document 1).

Furthermore, it has been recently reported that a combination use of an antiangiogenic drug and a chemotherapeutic agent shows a high effectiveness (Non-Patent Document 2).
Non-Patent Document 1: M. Iwata, et al., Retinoic acids exert direct effects on T cells to suppress Th1 development and enhance Th2 development via retinoic acid receptors. International Immunology, 15(8): pp.1017-1025, 2003.
Non-Patent Document 2: Sengupta, S., et al., Temporal atrgeting of tumor cells and neovasculature with a nanoscale delivery system. Nature, 436: pp.568-572, 2005.

### Disclosure of Invention

### Problems to be Solved by the Invention

As described above, since that retinoids cause various immune reactions and a combination use of an agent targeting tumor vessels and a chemotherapeutic agent shows a high effectiveness on tumor cells as a target, it is expected to reduce toxicity against normal cells in order to enhance specificity to tumor cells. However, at the same time, an administration method that does not act on normal cells but enhances cytotoxicity against tumor cells more than ever is highly expected.

Accordingly, it is an object of the present invention to provide a kit for cancer treatment and a pharmaceutical composition for cancer treatment for inhibiting the growth of tumors and cancers in mammals more than ever.

### Means for Solving the Problems

The present inventor has conducted intensive studies for solving the above-mentioned problems and has found that a synthetic retinoid is particularly useful for suppression of the growth of cancers and tumors and further found that a high effectiveness can be obtained by formulating the synthetic retinoid to a kit preparation that is sequentially used in a combination with another chemotherapeutic agent that is effective for reducing tumor size (debulking), and side effects of the kit preparation are milder compared to those in a combination use of only chemotherapeutic agents. The present invention has been thus completed.

That is, the present invention relates to the following (1) to (12):
(1) A kit for cancer treatment including a combination of two different drugs in a kit formulation, wherein a first drug contains a synthetic retinoid or a pharmaceutically acceptable organic or inorganic acid addition salt thereof, and a second drug contains a chemotherapeutic agent for cancer treatment;
(2) In the kit for cancer treatment according to the above (1), the synthetic retinoid is a benzoic acid derivative represented by the following formula (I): 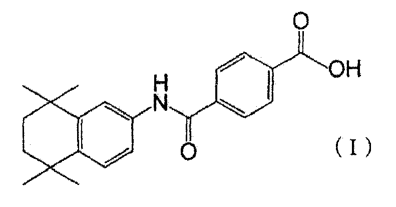 or a pharmaceutically acceptable organic or inorganic acid addition salt thereof;
(3) In the kit for cancer treatment according to the above (1) or (2), the synthetic retinoid is an agonist or an antagonist of a retinoic acid receptor (RAR α, β, or γ) or a retinoid receptor (RXR α, (β, or γ), or a pharmaceutically acceptable organic or inorganic acid addition salt thereof;
(4) In the kit for cancer treatment according to any of the above (1) to (3), the first drug contains a synthetic retinoid or a pharmaceutically acceptable organic or inorganic acid addition salt thereof at 0.5 to 30 mg per human or animal, and the second drug contains a chemotherapeutic agent at 1.0 to 1000 mg per human or animal;
(5) In the kit for cancer treatment according to any of the above (1) to (4), the chemotherapeutic agent is selected from the group consisting of DNA interactive agents, antimetabolites, tubulin interactive agents, anticancer antibiotics, enzyme inhibitors, growth-promoting-signal inhibitors, and anti-hormone agents;
(6) In the kit for cancer treatment according to the above (5), the chemotherapeutic agent is selected from the group consisting of steroids, asparaginases, hydroxyureas, cisplatins, cyclophosphamides, altretamine, bleomycins, dactinomycins, doxorubicins, etoposides, and teniposides;
(7) In the kit for cancer treatment according to the above (5), the chemotherapeutic agent is selected from the group consisting of methotrexate, fluorouracil, fluorodeoxyuridine, azacitidine, cytarabine, mercaptopurine, 6-thioguanine, pentostatin, and fludarabine;
(8) In the kit for cancer treatment according to the above (5), the chemotherapeutic agent is selected from the group consisting of vinca alkaloids and taxanes;
(9) In the kit for cancer treatment according to the above (5), the chemotherapeutic agent is selected from the group consisting of growth factor receptor tyrosine kinase inhibitors, cyclooxygenase-2 inhibitors, histone deacetylase inhibitors, and DNA methylation inhibitors;
(10) In the kit for cancer treatment according to the above (5), the chemotherapeutic agent is a steroid agent, an anti-estrogen agent, or an anti-androgen agent;
(11) In the kit for cancer treatment according to any of the above (1) to (10), the first drug contains a synthetic retinoid or a pharmaceutically acceptable organic or inorganic acid addition salt thereof at a dosage for 21 days or 28 days, and the second drug contains a chemotherapeutic agent at a dosage for treatment for 1 to 21 days or 28 days; and
(12) A pharmaceutical composition for cancer treatment including a first drug and a second drug in the kit according to any of the above (1) to (11).

### Advantageous Effect of the Invention

According to the present invention, a synthetic retinoid is first administered and then a chemotherapeutic agent is administered, and thereby the chemotherapeutic agent showing cytotoxicity can reduce or contract the size of a tumor mass more than ever. In addition, the method of simultaneously administering a synthetic retinoid and a chemotherapeutic agent is useful. Examples of cancers that are treated with the present invention include blood cancers such as acute myelocytic leukemia, acute lymphocytic leukemia, multiple myeloma, and non-Hodgkin's lymphoma; lung cancer; digestive cancers such as colon cancer, gastric cancer, liver cancer, pancreatic cancer, and bile duct cancer; breast cancer; prostate cancer; ovary cancer; and uterine cancer.

### Brief Description of Drawings

Fig. 1 is a graph showing a synergistic effect on growth-suppressing activity in a combination use of TM-411 and As₂O₃ for human acute promyelocytic leukemia cell line HL-60.
Fig. 2 is a graph showing a synergistic effect on growth-suppressing activity in a combination use of TM-411 and 5-AZ for human acute promyelocytic leukemia cell line HL-60.
Fig. 3 is a graph showing an additive effect and a synergistic effect on growth-suppressing activity in a combination use of TM-411 and Mel for human multiple myeloma cell line RPMI8226.
Fig. 4 is a graph showing a synergistic effect on growth-suppressing activity in a combination use of TM-411 and PSL for human multiple myeloma cell line RPMI8226.
Fig. 5 is a graph showing a synergistic effect on growth-suppressing activity in a combination use of TM-411 and DEX for human multiple myeloma cell line RPMI8226.
Fig. 6 is a graph showing a synergistic effect on growth-suppressing activity in a combination use of TM-411 and 5-AZ for human multiple myeloma cell line RPMI8226.
Fig. 7 is a graph showing an additive effect and a synergistic effect on growth-suppressing activity in a combination use of TM-411 and VPA for human hepatocellular carcinoma cell line JHH-7.

### Best Modes for Carrying Out the Invention

In the kit of the present invention, examples of the synthetic retinoid or a pharmaceutically acceptable organic or inorganic acid addition salt thereof contained in the first drug are preferably benzoic acid derivatives or pharmaceutically acceptable organic or inorganic acid addition salts thereof and particularly preferably a benzoic acid derivative represented by the following formula (I): 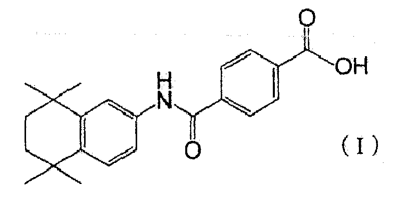 or a pharmaceutically acceptable organic or inorganic acid addition salt thereof.

The synthetic retinoid can be an agonist or an antagonist of a retinoic acid receptor (RAR α, β, or γ) or a retinoid receptor (RXR α, β, or γ), or a pharmaceutically acceptable organic or inorganic acid addition salt thereof.

In addition, in the kit of the present invention, examples of the DNA interactive agent as the chemotherapeutic agent contained in the second drug include alkylation agents such as cyclophosphamide, isophamide, melphalan, thiotepa, busulfan, carmustine, lomustine, cisplatin, and carboplatin; DNA topoisomerase I inhibitors such as camptothecine; and DNA topoisomerase II inhibitors such as etoposide, teniposide, daunorubicin, doxorubicin, and mitoxantrone, and are preferably orally available DNA interactive agents: cyclophosphamide, melphalan, cisplatin, camptothecine, etoposide, and doxorubicin.

Some of the antimetabolites as the chemotherapeutic agents inhibit DNA replication by inhibiting biosynthesis of deoxyribonucleoside triphosphate, which is the immediate precursor of DNA synthesis. Some of the antimetabolites are sufficiently similar to purine or pyrimidines to be able to substitute for them in the anabolic nucleotide pathways. In general, these analogs can then be substituted into the DNA and RNA instead of their normal counterparts. Examples of the antimetabolites useful herein include folate antagonists such as methotrexate and trimetrexate; pyrimidine antagonists such as 5-fluorouracil, fluorodeoxyuridine, Azacitidine, cytarabine, and floxuridine; purine antagonists such as mercaptopurine, 6-thioguanine, Fludarabine, and Pentostatin; and ribonucleotide reductase inhibitors such as hydroxyurea, and are preferably orally available methotrexate, 5-fluorouracil, cytarabine, and Fludarabine. In addition, examples of DNA methylation inhibitors include 5-aza-2'-deoxycytidine (hereinafter, abbreviated to "5-AZ").

The tubulin interactive agents as the chemotherapeutic agents act by binding to specific sites on tubulin, namely, a protein that polymerizes to form cellular microtubules. Examples of the tubulin interactive agents include vincristine and vinblastine, which both are alkaloids, and Paclitaxel and docetaxel, and are preferably orally available Paclitaxel and docetaxel.

Examples of the anticancer antibiotic as the chemotherapeutic agent include bleomycin having a DNA-cleaving activity, mitomycin acting as a bioreduction alkylation agent, and doxorubicin acting as a DNA-intercalating agent. Doxorubicin is preferred. In addition, valproic acid used as an antiepileptic agent and arsenous acid, which inhibits membrane permeability of mitochondria, can be also used as the anticancer antibiotic as the chemotherapeutic agents.

Examples of the enzyme inhibitor as the chemotherapeutic agent include cyclooxygenase-2 inhibitors such as celecoxib, rofecoxib, and curcumin; histone deacetylase inhibitors such as valproic acid, FK-228, and MS-275; farnesyl transferase inhibitors such as R115777 and BMS214662; and DNA methylation inhibitors such as 5-AZ, and are preferably orally available celecoxib, valproic acid, FK-228, and 5-AZ.

Examples of the growth-promoting-signal inhibitor as the chemotherapeutic agent include EGFR tyrosine kinase inhibitors such as gefinitib and erlotinib; and Bcr-abl tyrosine kinase inhibitors such as imanitib, and are preferably orally available gefinitib and imanitib.

Luteinizing hormone-releasing hormone agents and gonadotropin-releasing hormone antagonists as the chemotherapeutic agents are mainly used for treatment of prostate cancer. These agents include leuprolide acetate and goserelin acetate. Examples of the anti-hormone agents include anti-estrogen agents such as Tamoxifen; anti-androgen agents such as Flutamide and bicalutamide; and anti-adrenal gland agents such as mitotane and aminogluthetimide, and are preferably orally available Tamoxifen, Flutamide, bicalutamide, and anti-adrenal gland agents. Examples of the steroids used in hormone therapy include dexamethasone, and examples of the hormone or anti-hormone antagonist include Prednisolone.

The first drug and the second drug in the kit of the present invention are useful for inhibiting the growth of cancers and other tumors in human or animals by administering effective doses of them orally, rectally, topically, or parenterally, or by injection to a vein or a tumor. Each drug of such a unit may be a solid such as pills, tablets, capsules, or liposome; gel; or a liquid that is suitable for oral, rectal, topical, intravascular, or parental administration, or for injection to the inside or near tumor cells (refer to Cancer Chemotherapy Handbook, 2nd edition, pp. 15-34, Appleton & Lange (Connecticut, 1994)).

The kit of the present invention preferably includes the first drug containing a synthetic retinoid or a pharmaceutically acceptable organic or inorganic acid addition salt thereof at a dosage for 21 days or 28 days and the second drug containing a chemotherapeutic agent at a dosage for treatment for 1 to 21 days or 28 days.

Specifically, when such a kit is used for therapy, effective amounts can be appropriately determined according to the therapy of a specific cancer or tumor type to be treated, and the route of applying the effective amount also varies depending on the tumor to be treated. Preferably, a synthetic retinoid is administered first to reduce the size of a cancer or tumor mass. In general, it takes from two to eight weeks. The reduction of the tumor or cancer cells in size is less than 50% of the original. Then, when the reduction of the tumor in size is observed, a chemotherapeutic agent is administered. Since the synthetic retinoid is relatively safe, it can be administered for from two weeks to one year, if necessary, for maintaining the effectiveness for suppressing cancer regrowth.

### Examples

The present invention will now be described with reference to Examples. It should be understood that the following Examples are illustrative and are not intended to restrict the scope of the present invention.

### Example 1.

### (Effects of synthetic retinoid, taxotere (DXL), and a combination use of the synthetic retinoid and taxotere (DXL) on human breast cancer Br-10 in nude mice)

Tamibarotene (hereinafter, simply abbreviated to "TM-411") was used as a synthetic retinoid and was also used in the following other Examples. Taxotere (hereinafter, abbreviated to "DXL"), which is a general name of that manufactured by sanofi-aventis K.K., was used as a chemotherapeutic agent.

A tumor cell mass of human breast cancer Br-10 was subcutaneously transplanted in dorsal portions of 6-week-old BALB/cAJcl-nu nude mice through a trocar. When the tumor volume reached 100 to 300 mm³, the administration was started. TM-411 was orally administered once a day at a dose of 1 mg/kg for 28 days every day. DXL was intravenously administered from the first day at a dose of 7.5 mg/kg every 4 days three times.

In a combination use group of TM-411 and DXL, each agent was administered at the same amount and the same administration route as those in the single administration. Tumor diameters were measured every day from the day of the start of the administration (the 1st day) to the next day of the completion of the administration (the 29th day), and effect on suppression of the growth was investigated. Table 1 shows the results. In a control group, physiological saline was administered at the same amount and the same administration route as those in the single administration of each agent.

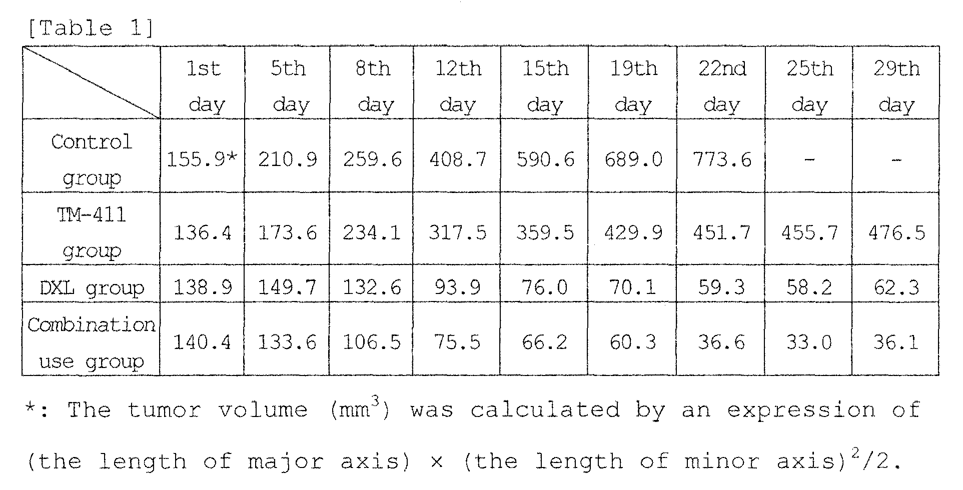

TM-411, DXL, or a combination of TM-411 and DXL was administered to the nude mice transplanted with human breast cancer Br-10. Though the growth of the tumor was significantly suppressed by the administration of TM-411 or DXL, the effect of the combination use of TM-411 and DXL exceeded the effect of single administration of each agent and showed a synergistic effect.

### Example 2

### (Effects of TM-411, 5-fluorouracil (5-FU), and a combination use of TM-411and 5-FUon human liver cancer JHH-7in nude mice)

Fluorouracil(hereinafter, abbreviated to "5-FU"), which is a general name of that manufactured by Kyowa Hakko Kogyo Co., Ltd., was used as a chemotherapeutic agent.

A tumor cell mass of human liver cancer JHH-7 was subcutaneously transplanted in dorsal portions of 6-week-old BALB/cAJcl-nu nude mice through a trocar. When the tumor volume reached 100 to 300 mm³,the administration was started. TM-411 was orally administered once a day at a dose of 3 mg/kg for 20 days every day. 5-FU was orally administered at a dose of 15.2 mg/kg for 5 days every day from the 8th day to the 12th day.

In a combination use group of TM-411 and 5-FU, each agent was administered at the same amount and the same administration route as those in the single administration. Tumor diameters were measured every day from the day of the start of the administration (the 1st day) to the next day of the completion of the administration (the 21st day), and effect on suppression of the growth was investigated. Table 2 shows the results. In a control group, physiological saline was administered at the same amount and the same administration route as those in the single administration of each agent.

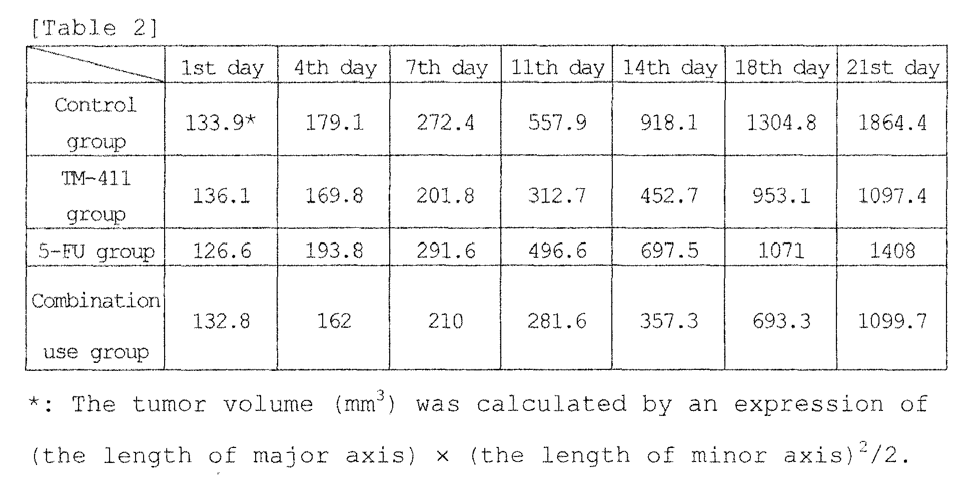

TM-411,5-FU, or a combination of TM-411 and 5-FU was administered to the nude mice transplanted with human liver cancer JHH-7.Though the growth of the tumor was suppressed by TM-411 or 5-FU, the effect of the combination use of TM-411 and 5-FU exceeded the effect of single administration of each agent and showed a synergistic effect.

### Example 3

### (Effects of TM-411,doxorubicin (ADR), and a combination use of TM-411 and ADR on human liver cancer HePG2 in nude mice)

Doxorubicin (hereinafter, abbreviated to "ADR"), which is a general name of that manufactured by Kyowa Hakko Kogyo Co., Ltd., was used as a chemotherapeutic agent.

About 2 x 2 x 2 mm of a tumor cell mass of human liver cancer HePG2 was subcutaneously transplanted in dorsal portions of 6-week-old BALB/cAJcl-nu nude mice through a trocar. The administration was started on the day (defined as the first day) two days after the transplantation. TM-411 was orally administered at a dose of 1 mg/kg for 20 days every day from the first day. ADR was intravenously administered at a dose of 5 mg/kg once on the first day.

In a combination use group of TM-411 and ADR, each agent was administered at the same amount and the same administration route as those in the single administration. Tumor diameters were measured every day from the day of the start of the administration (the 1st day) to the day of the completion of the administration (the 20th day), and effect on suppression of the growth was investigated. Table 3 shows the results. In a control group, physiological saline was administered at the same amount and the same administration route as those in the single administration of each agent.

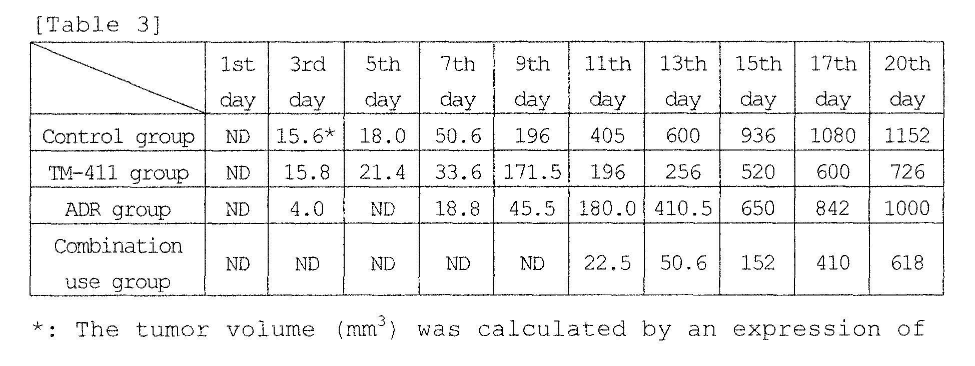

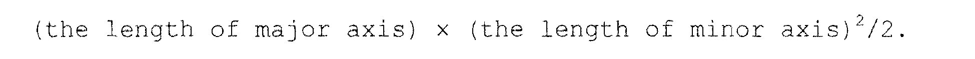

Both TM-411 and ADR suppressed the growth of the tumor by administering TM-411 or ADR to the nude mice transplanted with human liver cancer HePG2. The effect of the combination use of TM-411 and ADR exceeded the effect of single administration of each agent and showed a synergistic effect.

### Example 4

### (Effects of TM-411, prednisolone, and a combination use of TM-411 and prednisolone on human multiple myeloma U266 in NOG mice)

Prednisolone (hereinafter, abbreviated to "PSL"), which is a general name of that manufactured by Shionogi & Co., Ltd., was used as a chemotherapeutic agent.

Seven-week-old NOD/SCID/γc^{null} (NOG) mice were divided into five groups of ten mice each (nine mice only in a control group), and 2 x 16⁶ cells of multiple myeloma cell line U266 were transplanted to each mouse via tail vein injection (the 0th day). The first group was used as a non-treated control group; in the second group, 1 mg/kg of TM-411 was orally administered for 28 days from the first day; in the third group, 3 mg/kg of TM-411 was orally administered for 28 days from the first day; in the fourth group, 7.5 mg/kg of PSL was orally administered for 8 days from the 21st day; and in the fifth group, 1 mg/kg of TM-411 was orally administered for 28 days from the first day and 7.5 mg/kg of PSL was orally administered in addition to the TM-411 for 8 days from the 21st day. Blood concentration of human IgE was measured 6 weeks after the transplantation of U266. Table 4 shows the results.

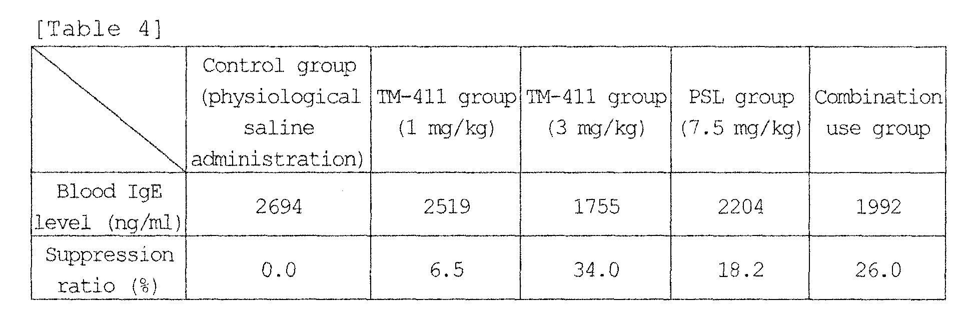

As shown in Table 4, both TM-411 and PSL decreased blood concentration of human IgE by administering TM-411 or PSL to the NOG mice transplanted with human multiple myeloma U266. The combination use of TM-411 and PSL showed a synergistic effect on the decrease of the blood concentration of human IgE.

### Example 5

### (Effect of a combination use of TM-411 and arsenous acid on human acute promyelocytic leukemia cell line HL-60)

Arsenous acid (hereinafter, abbreviated to "As₂O₃") manufactured by Sigma-aldrich Japan K.K. was used as a chemotherapeutic agent.

Human acute promyelocytic leukemia cell line HL-60 cells were cultured in Iscove's modified Dulbecco's medium

(GIBCO Laboratories) containing 20% bovine fetal serum, and then TM-411and As₂O₃ were added thereto. The synergistic effect on the growth-suppressing activity was verified by MTT (5% thiazolyl blue tetrazolium bromide) assay.

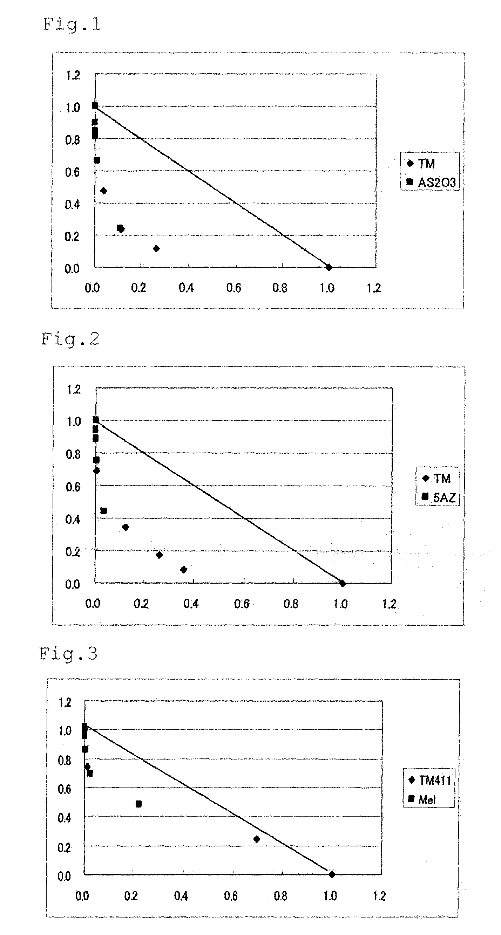

In Fig. 1, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of As₂O₃ is assumed to be 1. Points on the straight line connecting the points of 1.0 on both axes mean an additive effect, and points on the underside of the line mean a synergistic effect. As shown in Fig. 1, it was observed that the combination use of TM-411 and As₂O₃ had a synergistic effect on the growth-suppressing activity in human acute promyelocytic leukemia cell line HL-60.

### Example 6

### (Effect of a combination use of TM-411 and 5-AZ, a DNA methylation inhibitor, on human acute promyelocytic leukemia cell line HL-60)

5-AZ manufactured by CALBIOCHEM was used as a DNA methylation inhibitor.

The synergistic effect on the growth-suppressing activity in the addition of TM-411 and 5-AZ was investigated by the same technique as that in Example 5.

In Fig. 2, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of 5-AZ is assumed to be 1. Points on the straight line connecting the points of 1.0 on both axes mean an additive effect, and points on the underside of the line mean a synergistic effect. As shown in Fig. 2, it was observed that the combination use of TM-411 and 5-AZ had a synergistic effect on the growth-suppressing activity in human acute promyelocytic leukemia cell line HL-60.

### Example 7

### (Effect of a combination use of TM-411 and melphalan on human multiple myeloma cell line RPMI8226)

Melphalan (hereinafter, abbreviated to "Mel") was used as a chemotherapeutic agent.

Human multiple myeloma cell line RPMI8226 cells were cultured in RPMI1640 medium containing 10% bovine fetal serum, and then TM-411 and Mel were added thereto. The synergistic effect in the growth-suppressing activity was verified.

In Fig. 3, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of Mel is assumed to be 1. Points on the straight line connecting the points of 1.0 on both axes mean an additive effect, and points on the underside of the line mean a synergistic effect. As shown in Fig. 3, it was observed that the combination use of TM-411 and Mel had an additive to synergistic effect on the growth-suppressing activity in human multiple myeloma cell line RPMI8226.

### Example 8

### (Effect of a combination use of TM-411 and PSL on human multiple myeloma cell line RPMI8226)

PSL was used as a chemotherapeutic agent.

The synergistic effect on the growth-suppressing activity in the addition of TM-411 and PSL was verified by the same technique as that in Example 7.

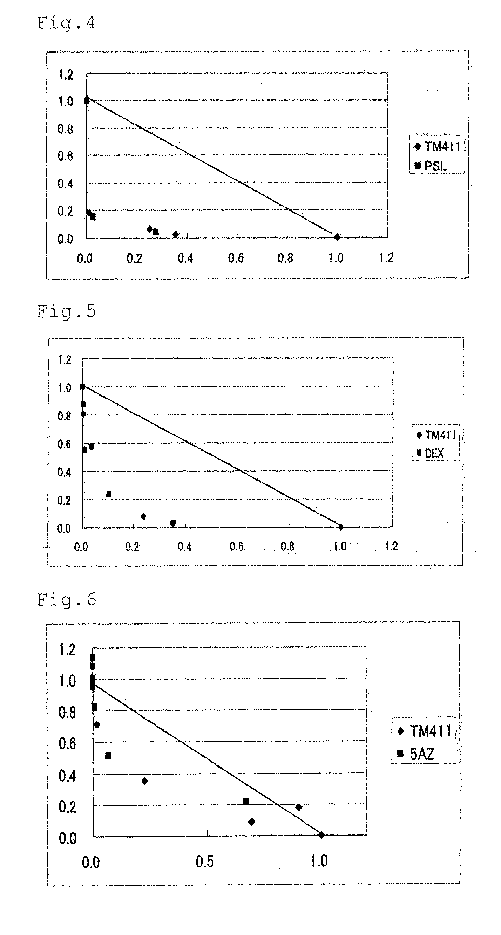

In Fig. 4, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of PSL is assumed to be 1. Points on the straight line connecting the points of 1.0 on both axes mean an additive effect, and points on the underside of the line mean a synergistic effect. As shown in Fig. 4, it was observed that the combination use of TM-411 and PSL had a synergistic effect on the growth-suppressing activity in human multiple myeloma cell line RPMI8226.

### Example 9

### (Effect of a combination use of TM-411 and dexamethasone on human multiple myeloma cell line RPMI8226)

Dexamethasone (hereinafter, abbreviated to "DEX") was used as a chemotherapeutic agent.

The synergistic effect on the growth-suppressing activity in the addition of TM-411 and DEX was verified by the same technique as that in Example 7.

In Fig. 5, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of DEX is assumed to be 1. Points on the straight line connecting the points of 1.0 on both axes mean an additive effect, and points on the underside of the line mean a synergistic effect. As shown in Fig. 5, it was observed that the combination use of TM-411 and DEX had a synergistic effect on the growth-suppressing activity in human multiple myeloma cell line RPMI8226.

### Example 10

### (Effect of a combination use of TM-411 and 5-AZ, a

methylation inhibitor, on human multiple myeloma cell line RPMI8226)

5-AZ was used as a methylation inhibitor.

The synergistic effect on the growth-suppressing activity in the addition of TM-411 and 5-AZ was verified by the same technique as that in Example 7.

In Fig. 6, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of 5-AZ is assumed to be 1. Points on the straight line connecting the points of 1.0 on both axes mean an additive effect, and points on the underside of the line mean a synergistic effect. As shown in Fig. 6, it was observed that the combination use of TM-411 and 5-AZ had a synergistic effect on the growth-suppressing activity in human multiple myeloma cell line RPMI8226.

### Example 11

### (Effect of a combination use of TM-411 and valproic acid on human hepatocellular carcinoma cell line JHH-7)

Valproic acid (hereinafter, abbreviated to "VPA") was used as a chemotherapeutic agent.

The additive and synergistic effect on the growth-suppressing activity in the addition of TM-411 and VPA was verified by the same technique as that in Example 7.

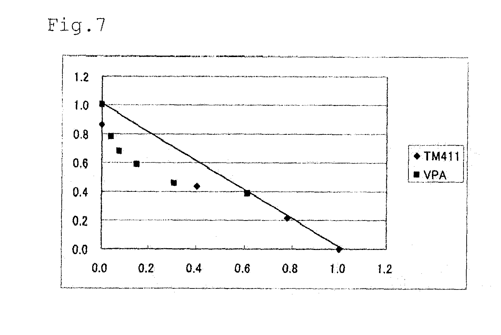

In Fig. 7, the horizontal axis denotes the ratio when the concentration at a point of ED₅₀ in the growth suppression curve of TM-411 is assumed to be 1, and the vertical axis denotes the ratio when the concentration at a point of ED₅₀ in the growth suppression curve of VPA is assumed to be 1. Points on the straight line connecting the points of 1.0 on both axes mean an additive effect, and points on the underside of the line mean a synergistic effect. As shown in Fig. 7, it was observed that the combination use of TM-411 and VPA had an additive to synergistic effect on the growth-suppressing activity in human hepatocellular carcinoma cell line JHH-7.

## Claims

1. A kit comprising a combination of a retinoid therapeutic agent, wherein the retinoid is represented by formula (I): 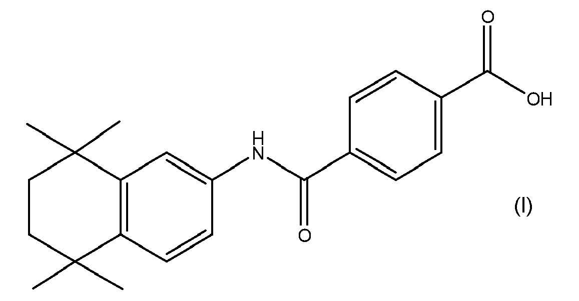 or a pharmaceutically acceptable organic or inorganic acid addition salt thereof, and the chemotherapeutic agent is selected from tubulin interactive agents.

2. The kit according to claim 1, wherein the tubulin interactive agents are selected from vinca alkaloids and taxanes.

3. The kit according to any one of claims 1-2, wherein the tubulin interactive agents are selected from taxanes, preferably Taxotere™.

4. The kit according to any one of claims 1-3, wherein the retinoid is in a dose of 0.5 to 30 mg per human or animal, and the chemotherapeutic agent is in a dose of 1.0 to 1000 mg per human or animal.

5. The kit according to any one of claims 1-4, wherein the retinoid is in a dosage for 21 days or 28 days, and the chemotherapeutic agent is in a dosage for treatment for 1 to 21 days or 28 days.

6. A pharmaceutical composition, comprising a combination of the retinoid and the chemotherapeutic agent according to any one of claims 1-5.

7. The kit of any one of claims 1-5 or the pharmaceutical composition of claim 6, for use in treating cancer.

8. The kit or pharmaceutical composition according to claim 7, wherein the cancer is a tumor.

9. The kit or pharmaceutical composition according to claim 7, wherein the cancer is a blood cancer.

10. The kit or pharmaceutical composition according to claim 9, wherein the blood cancer is a leukemia, a multiple myeloma, or a non-Hodgkin's lymphoma.

11. The kit or pharmaceutical composition according to claim 9 or 10, wherein the blood cancer is a leukemia.

12. The kit or pharmaceutical composition according to any one of claims 9-11, wherein the blood cancer is an acute myelocytic leukemia or an acute lymphocytic leukemia.

13. The kit or pharmaceutical composition according to any one of claims 9-12, wherein the blood cancer is an acute promyelocytic leukemia.

14. The kit or pharmaceutical composition according to claim 7, wherein the cancer is selected from lung cancer, colon cancer, gastric cancer, liver cancer, pancreatic cancer, bile duct cancer, breast cancer, prostate cancer, ovary cancer, and uterine cancer.

15. The kit or pharmaceutical composition according to claim 14, wherein the cancer is a lung cancer.
